# EUROPEAN PATENT APPLICATION

(11) **EP 4 155 406 A1**
(43) Date of publication of application: **29.03.2023**
(21) Application number: 21807863.2
(22) Date of filing: 14.05.2021
(51) Int. Cl.: C12N 15/50, C07K 14/165, A61K 39/215, A61P 31/14

(54) **MRNA OR MRNA COMPOSITION, AND PREPARATION METHOD THEREFOR AND APPLICATION THEREOF**

(30) Priority: 18.05.2020 CN 202010419984
(71) Applicant: Cansino (Shanghai) Biotechnologies Co.,Ltd, Shanghai 201208 (CN)
(72) Inventor: ZHU, Tao, Tianjin 300457 (CN); WANG, Haomeng, Tianjin 300457 (CN); LI, Jin, Tianjin 300457 (CN); YAN, Qiaoling, Tianjin 300457 (CN); SHEN, Chunlin, Tianjin 300457 (CN); SHAO, Zhongqi, Tianjin 300457 (CN); LI, Junqiang, Tianjin 300457 (CN); YU, Xuefeng, Tianjin 300457 (CN); CHAO, Shoubai, Tianjin 300457 (CN)
(74) Representative: HGF
(86) International application number: PCT/CN2021/093741
(87) International publication number: WO 2021/233213

(57) **Abstract**

Provided are an mRNA or an mRNA composition, and an mRNA vaccine comprising the mRNA or the mRNA composition. The mRNA or the mRNA composition comprises an mRNA sequence encoding an S protein of a novel coronavirus SARS-CoV-2 or a variant thereof, and an mRNA sequence encoding an RBD in the S protein or a variant thereof. Further provided are the applications of the mRNA or the mRNA composition, and the mRNA vaccine comprising the mRNA or the mRNA composition in preparation of a medication for preventing and/or treating a disease caused by a novel coronavirus SARS-CoV-2 infection.

## Description

### TECHNICAL FIELD

The present invention relates to the technical field of vaccine development and production, and in specific to a vaccine comprising an mRNA sequence encoding a spike protein (S protein) of SARS-CoV-2 or a variant thereof and an mRNA sequence encoding a receptor binding domain (RBD) in the S protein or the variant thereof. The present invention further relates to a composition comprising one or two mRNAs, and use of the mRNA or the composition in preparing a medicament (particularly a vaccine) for preventing and/or treating SARS-CoV-2 infection.

### BACKGROUND

Coronaviruses are non-segmented, single-stranded, positive-strand RNA viruses belonging to the subfamily *Orthocoronavirinae*, family *Coronaviridae*, order *Nidovirales. Orthocoronavirinae* is classified into four genera, *Alphacoronavirus*, *Betacoronavirus*, *Gammacoronavirus* and *Deltacoronavirus*, according to serotype and genomic characteristics. To date, 7 coronaviruses were found capable of infecting humans, including *Alphacoronavirus* 229E and NL63, and *Betacoronavirus* OC43, HKU1, Middle East respiratory syndrome-related coronavirus (MERSr-CoV), severe acute respiratory syndrome-related coronavirus (SARSr-CoV) and severe acute respiratory syndrome coronavirus 2 (SARS-CoV-2). Among these, the last three may lead to severe human diseases and even death.

Coronaviruses are enveloped in round or oval particles, often polymorphic, typically 50-200 nm in diameter. The S protein is located on the surface of the virus to form a rod-shaped structure, and is one of main antigen proteins of the virus and a main gene for genotyping. The N protein encapsulates the viral genome and can be used as a diagnostic antigen. The interpretation of the physicochemical properties of coronaviruses is mostly derived from studies on SARS-CoV and MERS-CoV. Vaccines designed based on the full-length S protein of SARS-CoV were reported to induce a large amount of non-neutralizing antibodies, fail in challenge test in animal models and cause severe side effects such as increased morbidity, strong inflammatory response of liver tissue and liver damage (see "Evaluation of modified vaccinia virus ankara based recombinant SARS vaccine in ferrets", Vaccine 23, 2273-2279). Therefore, avoiding exposure of non-neutralizing epitopes with immune advantages in vaccine design is the basis for ensuring vaccine safety.

The RBDs of both SARS-CoV and MERS-CoV consist of two components: a highly similar core structure and a highly variable receptor binding motif (RBM). The difference in RBM allows SARS-CoV and MERS-CoV to recognize different receptors: SARS-CoV recognizes angiotensin-converting enzyme 2 (ACE2), while MERS-CoV recognizes dipeptidyl peptidase 4 (DPP4).

Vaccine platforms involved in the current development of SARS-CoV and MERS-CoV vaccines include viral vector vaccines, DNA vaccines, subunit vaccines, virus-like particle (VLP) vaccines, inactivated whole virus vaccines and attenuated vaccines.

Theoretically, inactivated whole virus vaccines that can be fast produced could respond to the epidemic outbreak of SARS-CoV-2. However, the culture of the virus requires biosafety level three laboratories, which is hardly satisfied by general vaccine enterprises; also, the vaccines may poses safety issues, and allergic pathological phenomena in lungs were reported in mouse model challenge tests for inactivated whole virus vaccines of SARS-CoV and MERS-CoV in research and development (see "Immunization with inactivated middle east respiratory syndrome coronavirus vaccine leads to lung immunopathology on challenge with live virus", Hum. Vaccin. Immunother. 12, 2351-2356). Therefore, inactivated whole virus vaccine is not the optimal choice for developing COVID-19 vaccines.

Because aged and immunosuppressed cohorts are also hosts of SARS-CoV-2, and the vaccine prepared by the attenuated live vaccine platform is not suitable for such individuals, the vaccine is not suitable for the research and development of COVID-19 vaccines.

Both DNA vaccines and viral vector vaccines deliver DNA to the cells of the vaccinees for expression. Although no integration and recombination into the genome have been reported, for example, for vaccines based on adenoviral vectors, this possibility cannot be completely ruled out and there is still a safety risk. Subunit vaccines and VLP vaccines require establishment of methods for optimizing expression and for purification, and generally require appropriate adjuvants. Thus, such vaccines may require years of research, and hardly respond to rapidly developing epidemics.

In recent years, with the breakthrough of related technologies in the field of RNA molecules, mRNA vaccines have been studied to some extent on various infectious diseases such as influenza virus, Ebola virus and Zika virus. mRNA vaccines transmit mRNA to cells, which express and produce proteins to provide immune protection for the body. Compared with the conventional recombinant protein vaccines, inactivated vaccines and attenuated vaccines, mRNA vaccines have simple preparation procedures and great significance for controlling infectious diseases. In addition, mRNA vaccines are more resistant to high temperature and more stable than conventional recombinant vaccines. Meanwhile, mRNA vaccines are able to elicit strong CD4⁺ or CD8⁺ T cell responses, and unlike DNA immunization, mRNA vaccines are able to produce antibodies in animals by one or two low-dose vaccinations.

Therefore, the present invention provides a safe and reliable mRNA vaccine, and avoids the defects of other vaccine platforms.

### SUMMARY

In a first aspect of the present invention, provided is an mRNA or mRNA composition, comprising: an mRNA sequence encoding an S protein (spike protein) of SARS-CoV-2 or a variant thereof, and an mRNA sequence encoding an RBD (receptor binding domain) in the S protein or a variant thereof.

The mRNA sequence encoding the S protein of SARS-CoV-2 or the variant thereof and the mRNA sequence encoding the RBD in the S protein or the variant thereof are derived from the same SARS-CoV-2 mutant or different SARS-CoV-2 mutants.

Preferably, the S protein or the variant thereof comprises a wild-type full-length S protein or a full-length S protein fixed in a pre-fusion conformation.

More preferably, for stabilizing the conformation, the full-length S protein fixed in the pre-fusion conformation comprises a mutation at positions 682RRAR685 and/or a mutation at positions 986KV987, such that the S protein is fixed in the pre-fusion conformation. Most preferably, the full-length S protein fixed in the pre-fusion conformation is obtained by mutating 682RRAR685 of the wild-type full-length S protein to GSAG and/or 986KV987 to PP.

Also, the technical schemes disclosed herein are supported in part by the disclosures in the prior art, for example, by replacing one or two amino acids with proline in the first heptad repeat or close to the first heptad repeat can effectively stabilize the conformation (see U.S. Patent Application No. 20200061185 entitled PREFUSION CORONAVIRUS SPIKE PROTEINS AND THEIR USE).

In one specific embodiment of the present invention, the wild-type full-length S protein comprises an amino acid sequence set forth in SEQ ID NO: 1 or an amino acid sequence having 70%, 75%, 80%, 85%, 90%, 95% or 99% identity to SEQ ID NO: 1.

In one specific embodiment of the present invention, the full-length S protein fixed in the pre-fusion conformation comprises an amino acid sequence set forth in SEQ ID NO: 2 or SEQ ID NO: 15 or an amino acid sequence having 70%, 75%, 80%, 85%, 90%, 95% or 99% identity to SEQ ID NO: 2 or 15.

Preferably, the S protein or the variant thereof does not comprise a signal peptide, comprises a signal peptide of the wild-type S protein or comprises a signal peptide of the wild-type S protein and a preceding strong signal peptide; preferably, the strong signal peptide is a signal peptide of tissue plasminogen activator (tPA) or a signal peptide of serum immunoglobulin E (IgE).

In one specific embodiment of the present invention, a nucleotide sequence encoding a wild-type 2019-nCoV S protein without signal peptide is set forth in SEQ ID NO: 6.

In one specific embodiment of the present invention, the RBD comprises an amino acid sequence set forth in SEQ ID NO: 3 or SEQ ID NO: 13 or an amino acid sequence having 70%, 75%, 80%, 85%, 90%, 95% or 99% identity to SEQ ID NO: 3 or 13.

Preferably, the RBD or the variant thereof does not comprise a signal peptide, comprises a signal peptide of the wild-type S protein or comprises a signal peptide of the wild-type S protein and a preceding strong signal peptide; preferably, the strong signal peptide is a signal peptide of tissue plasminogen activator (tPA) or a signal peptide of serum immunoglobulin E (IgE).

In one specific embodiment of the present invention, a nucleotide sequence encoding a wild-type SARS-CoV-2 RBD with IgE signal peptide is set forth in any one of SEQ ID NOs: 7 and 9-11. Preferably, the mRNA is a monocistronic, bicistronic or polycistronic mRNA. The bicistronic or polycistronic mRNA is an mRNA comprising two or more coding regions.

Preferably, the mRNA sequence encoding the S protein of SARS-CoV-2 or the variant thereof and the mRNA sequence encoding the RBD in the S protein or the variant thereof are two separate mRNA sequences or are ligated in one mRNA sequence.

More preferably, the ligation order for the one mRNA sequence from 5' to 3' is: the mRNA sequence encoding the S protein of SARS-CoV-2 or the variant thereof to the mRNA sequence encoding the RBD in the S protein or the variant thereof, or the mRNA sequence encoding the RBD in the S protein or the variant thereof to the mRNA sequence encoding the S protein of SARS-CoV-2 or the variant thereof. Still more preferably, the mRNA sequence encoding the S protein of SARS-CoV-2 or the variant thereof and the mRNA sequence encoding the RBD in the S protein or the variant thereof are ligated via an internal ribosome entry site (IRES).

The IRES can be used to separate the two coding regions.

In one specific embodiment of the present invention, the IRES sequence includes, but is not limited to, picornavirus (e.g., FMDV), pestivirus (e.g., CFFV), poliovirus (e.g., PV), encephalomyocarditis virus (e.g., ECMV), foot-and-mouth disease virus (e.g., FMDV), hepatitis C virus (e.g., HCV), classical swine fever virus (e.g., CSFV), mouse leukoma virus (e.g., MLV), simian immunodeficiency virus (e.g., SIV) and cricket paralysis virus (e.g., CrPV).

Preferably, the mRNA or mRNA composition further comprises a 5' cap, a 5' non-coding region and a poly A tail.

More preferably, the mRNA or mRNA composition further comprises one or a combination of two or more of a 5' conserved sequence element, an RNA replicase coding region, a subgenomic promoter, a 3' conserved sequence element and a 3' non-coding region.

Preferably, the mRNA is a conventional mRNA, a self-amplifying mRNA or a trans-amplifying mRNA.

In one specific embodiment of the present invention, the mRNA is a conventional mRNA, i.e., the mRNA further comprises the 5' cap, the 5' non-coding region, the 3' non-coding region and/or the polyA tail in addition to the mRNA sequence encoding the S protein of SARS-CoV-2 or the variant thereof and the mRNA sequence encoding the RBD in the S protein or the variant thereof. In one specific embodiment of the present invention, the mRNA is a self-amplifying mRNA, i.e., the mRNA further comprises the 5' cap, the 5' conserved sequence element, the RNA replicase coding region, the subgenomic promoter, the 3' conserved sequence element and the polyA tail in addition to the mRNA sequence encoding the S protein of SARS-CoV-2 or the variant thereof and the mRNA sequence encoding the RBD in the S protein or the variant thereof. Available RNA replicase coding regions include, but are not limited to, alphavirus (e.g., SFV), picornavirus (e.g., FMDV), flavivirus (e.g., DENV), paramyxovirus (e.g., HMPV) and calicivirus (e.g., NV). In one specific embodiment of the present invention, the mRNA is a trans-amplifying mRNA, i.e., the mRNA encoding the target gene comprises the 5' cap, the 5' conserved sequence element, the subgenomic promoter, the 3' conserved sequence element and the polyA tail in addition to the mRNA sequence encoding the S protein of SARS-CoV-2 or the variant thereof and the mRNA sequence encoding the RBD in the S protein or the variant thereof; the RNA replicase is encoded by a single conventional mRNA.

In one specific embodiment of the present invention, the mRNA or mRNA composition comprises an mRNA sequence consisting of the mRNA sequence encoding the S protein of SARS-CoV-2 or the variant thereof and the mRNA sequence encoding the RBD in the S protein or the variant thereof selected from any one of the following:
A) consisting of the 5' cap, the 5' non-coding region, the mRNA sequence encoding the S protein of SARS-CoV-2 or the variant thereof, the mRNA sequence encoding the RBD in the S protein or the variant thereof, the 3' non-coding region and the polyA tail;
B) consisting of the 5' cap, the 5' non-coding region, the mRNA sequence encoding the RBD in the S protein or the variant thereof, the mRNA sequence encoding the S protein of SARS-CoV-2 or the variant thereof, the 3' non-coding region and the polyA tail;
C) consisting of the 5' cap, the 5' non-coding region, the mRNA sequence encoding the S protein of SARS-CoV-2 or the variant thereof, the internal ribosome entry site (IRES), the mRNA sequence encoding the RBD in the S protein or the variant thereof, the 3' non-coding region and the poly A tail;
D) consisting of the 5' cap, the 5' non-coding region, the mRNA sequence encoding the RBD in the S protein or the variant thereof, the IRES, the mRNA sequence encoding the S protein of SARS-CoV-2 or the variant thereof, the 3' non-coding region and the polyA tail;
E) consisting of the 5' cap, the 5' conserved sequence element, the RNA replicase coding region, the subgenomic promoter, the mRNA sequence encoding the RBD in the S protein or the variant thereof, the mRNA sequence encoding the S protein of SARS-CoV-2 or the variant thereof, the 3' conserved sequence element and the polyA tail;
F) consisting of the 5' cap, the 5' conserved sequence element, the RNA replicase coding region, the subgenomic promoter, the mRNA sequence encoding the S protein of SARS-CoV-2 or the variant thereof, the mRNA sequence encoding the RBD in the S protein or the variant thereof, the 3' conserved sequence element and the polyA tail;
G) consisting of the 5' cap, the 5' conserved sequence element, the RNA replicase coding region, the subgenomic promoter, the mRNA sequence encoding the RBD in the S protein or the variant thereof, the IRES, the mRNA sequence encoding the S protein of SARS-CoV-2 or the variant thereof, the 3' conserved sequence element and the polyA tail; and
H) consisting of the 5' cap, the 5' conserved sequence element, the RNA replicase coding region, the subgenomic promoter, the mRNA sequence encoding the S protein of SARS-CoV-2 or the variant thereof, the IRES, the mRNA sequence encoding the RBD in the S protein or the variant thereof, the 3' conserved sequence element and the polyA tail.

In one specific embodiment of the present invention, the mRNA or mRNA composition comprises a combination of two mRNA sequences selected from any one of the following:
a) an mRNA consisting of the 5' cap, the 5' non-coding region, the mRNA sequence encoding the S protein of SARS-CoV-2 or the variant thereof, the 3' non-coding region and the polyA tail, combined with an mRNA consisting of the 5' cap, the 5' non-coding region, the mRNA sequence encoding the RBD in the S protein or the variant thereof, the 3' non-coding region and the polyA tail;
b) an mRNA consisting of the 5' cap, the 5' conserved sequence element, the RNA replicase coding region, the subgenomic promoter, the mRNA sequence encoding the RBD in the S protein or the variant thereof, the 3' conserved sequence element and the polyA tail, combined with an mRNA consisting of the 5' cap, the 5' conserved sequence element, the RNA replicase coding region, the subgenomic promoter, the mRNA sequence encoding the S protein of SARS-CoV-2 or the variant thereof, the 3' conserved sequence element and the polyA tail;
c) an mRNA consisting of the 5' cap, the 5' conserved sequence element, the subgenomic promoter, the mRNA sequence encoding the S protein of SARS-CoV-2 or the variant thereof, the IRES, the mRNA sequence encoding the RBD in the S protein or the variant thereof, the 3' conserved sequence element and the polyA tail, combined with an mRNA consisting of the 5' cap, the 5' non-coding region, the RNA replicase coding region, the 3' non-coding region and the polyA tail;
d) an mRNA consisting of the 5' cap, the 5' conserved sequence element, the subgenomic promoter, the mRNA sequence encoding the RBD in the S protein or the variant thereof, the IRES, the mRNA sequence encoding the S protein of SARS-CoV-2 or the variant thereof, the 3' conserved sequence element and the polyA tail, combined with an mRNA consisting of the 5' cap, the 5' non-coding region, the RNA replicase coding region, the 3' non-coding region and the polyA tail;
e) an mRNA consisting of the 5' cap, the 5' conserved sequence element, the subgenomic promoter, the mRNA sequence encoding the S protein of SARS-CoV-2 or the variant thereof, the mRNA sequence encoding the RBD in the S protein or the variant thereof, the 3' conserved sequence element and the polyA tail, combined with an mRNA consisting of the 5' cap, the 5' non-coding region, the RNA replicase coding region, the 3' non-coding region and the polyA tail; and
f) an mRNA consisting of the 5' cap, the 5' conserved sequence element, the subgenomic promoter, the mRNA sequence encoding the RBD in the S protein or the variant thereof, the mRNA sequence encoding the S protein of SARS-CoV-2 or the variant thereof, the 3' conserved sequence element and the polyA tail, combined with an mRNA consisting of the 5' cap, the 5' non-coding region, the RNA replicase coding region, the 3' non-coding region and the polyA tail. In one specific embodiment of the present invention, the mRNA or mRNA composition comprises an mRNA sequence comprising any one or a combination of two or more of SEQ ID NOs: 16-19.

Preferably, the mRNA or mRNA composition further comprises a cationic or polycationic compound.

Preferably, the cationic or polycationic compound is free or bound to the mRNA.

In one specific embodiment of the present invention, the form in which the cationic or polycationic compound is bound to the mRNA is selected in order to make the mRNA or mRNA composition more stable.

Preferably, the mRNA or mRNA composition further comprises a lipid.

More preferably, the lipid includes, but is not limited to, a liposome capable of promoting self-assembly into virus-sized particles (appr. 100 nm), a liposome allowing intracellular release of mRNA from endosomes, a liposome supporting a phospholipid bilayer structure and a liposome working as a stabilizer.

More preferably, the lipid may further comprise a PEGylated lipid to increase the half-life of LNPs (lipid nanoparticles).

In one specific embodiment of the present invention, the lipid comprises a cationic lipid, a PEGylated lipid, cholesterol, and/or a phospholipid.

The mRNA or mRNA composition disclosed herein can be a liposome, a lipid complex or a lipid nanoparticle. The liposome can be prepared in the following forms: 1,2-dioleyloxy-N,N-dimethylaminopropane (DODMA) liposome, 1,2-dilinoleyloxy-3-dimethylaminopropane (DLin-DMA) liposome or 2,2-dilinoleyl-4-(2-dimethylaminoethyl)-[1,3]-dioxolane (DLin-KC2-DMA) liposome. The lipid complex or lipid nanoparticle may be formed from a lipid selected from: DLin-DMA, DLin-K-DMA, 98N12-5, C12-200, DLin-MC3-DMA, DLin-KC2-DMA, DODMA, PLGA, PEG, PEG-DMG, PEGylated lipid and aminoalcohol lipid.

The mRNA or mRNA composition disclosed herein may further comprise a pharmaceutically acceptable excipient. The pharmaceutically acceptable excipient may be a carrier, a diluent, an adjuvant or adjuvant-encoding nucleotide sequence, a solubilizer, a binder, a lubricant, a suspending agent, a transfection promoter or the like. Such transfection promoter includes, but is not limited to, surfactants such as immune stimulating complexes, Freunds incomplete adjuvant, LPS analogs (e.g., monophosphoryl lipid A), muramyl peptide, benzoquinone analogs, squalene, hyaluronic acid, lipids, liposomes, calcium ion, viral proteins, cations, polycations (e.g., poly-L-glutamic acid (LGS)), nanoparticles or other known transfection promoters. The nucleotide sequence of the coding adjuvant is a nucleotide sequence of at least one adjuvant as follows: GM-CSF, IL-17, IFNg, IL-15, IL-21, anti-PD1/2, lactoferrin, protamine, IL-1, IL-2, IL-3, IL-4, IL-5, IL-6, IL-7, IL-8, IL-9, IL-10, IL-12, INF-α, INF-γ, Lymphotoxin-α, hGH, MCP-1, MIP-1a, MIP-1p, IL-8, RANTES, L-selectin, P-selectin, E-selectin, CD34, GlyCAM-1, MadCAM-1, LFA-1, VLA-1, Mac-1, pl50.95, PECAM, ICAM-1, ICAM-2, ICAM-3, CD2, LFA-3, M-CSF, CD40, CD40L, vascular growth factor, fibroblast growth factor, nerve growth factor, vascular endothelial growth factor, Apo-1, p55, WSL-1, DR3, TRAMP, Apo-3, AIR, LARD, NGRF, DR4, DR5, KILLER, TRAII,-R2, TRICK2, DR6, caspase ICE, Fos, c-jun, Sp-1, Ap-1, Ap-2, p38, p65Rel, MyD88, IRAK, TRAF6, IkB, inactive NIK, SAP K, SAP-1, JNK, NFkB, Bax, TRAIL, TRAILrec, TRAILrecDRC5, TRAIL-R3, TRAIL-R4, RANK, RANK LIGAND, Ox40, Ox40LIGAND, NKG2D, MICA, MICB, NKG2A, NKG2B, NKG2C, NKG2E, NKG2F, TAP1, TAP2 and functional fragments thereof.

In a second aspect of the present invention, provided is an mRNA vaccine comprising the mRNA or mRNA composition disclosed herein.

Preferably, in mRNA vaccine, the sequence encoding the S protein of SARS-CoV-2 or the variant thereof and the sequence encoding the RBD in the S protein or the variant thereof are derived from different SARS-CoV-2 mutants, thus immunizing the body to produce cross protection against different SARS-CoV-2 mutants. In one specific embodiment of the present invention, in the mRNA vaccine, the sequence encoding the wild-type SARS-CoV-2 RBD with IgE signal peptide contains mutations K417N, E484K and N501Y of the 501Y.V2 strain, which preferably comprises an amino acid sequence set forth in SEQ ID NO: 13; the sequences encoding the full-length S protein fixed in the pre-fusion conformation with a mutation to GSAG at 682RRAR685 and a mutation to PP at 986KV987 is derived from Wuhan-Hu-1 isolate, which contains L18F, D80A, D215G, L242-L244 deletions (L242-244del), R246I, K417N, E484K, N501Y and A701V of the 501Y.V2 strain, and preferably comprises an the amino acid sequence set forth in SEQ ID NO: 15.

In one specific embodiment of the present invention, the mRNA vaccine comprises the mRNA sequence encoding the S protein of SARS-CoV-2 or the variant thereof and the mRNA sequence encoding the RBD in the S protein or the variant thereof, wherein the S protein of SARS-CoV-2 or the variant thereof has an amino acid sequence set forth in SEQ ID NO: 15, and the RBD in the S protein or the variant thereof is set forth in SEQ ID NO: 13.

In another specific embodiment of the present invention, the mRNA vaccine comprises the mRNA sequence encoding the S protein of SARS-CoV-2 or the variant thereof and the mRNA sequence encoding the RBD in the S protein or the variant thereof, wherein the S protein of SARS-CoV-2 or the variant thereof has an amino acid sequence set forth in SEQ ID NO: 2, and the RBD in the S protein or the variant thereof is set forth in SEQ ID NO: 13.

In another specific embodiment of the present invention, the mRNA vaccine comprises the mRNA sequence encoding the S protein of SARS-CoV-2 or the variant thereof and the mRNA sequence encoding the RBD in the S protein or the variant thereof, wherein the S protein of SARS-CoV-2 or the variant thereof has an amino acid sequence set forth in SEQ ID NO: 2, and the RBD in the S protein or the variant thereof is set forth in SEQ ID NO: 3. Preferably, in the mRNA vaccine, the mass ratio of the mRNA sequence encoding the S protein of SARS-CoV-2 or the variant thereof to the mRNA sequence encoding the RBD in the S protein or the variant thereof is (1-5):(1-5).

In one specific embodiment of the present invention, in the mRNA vaccine, the mass ratio of the mRNA sequence encoding the wild-type SARS-CoV-2 RBD with IgE signal peptide to the mRNA sequences encoding the full-length S protein fixed in the pre-fusion conformation with a mutation to GSAG at 682RRAR685 and a mutation to PP at 986KV987 is (1-2):(1-2). Preferably, the mRNA vaccine further comprises a cationic or polycationic compound. Preferably, the cationic or polycationic compound is free or bound to the mRNA.

In one specific embodiment of the present invention, the form in which the cationic or polycationic compound is bound to the mRNA is selected in order to make the mRNA vaccine more stable.

Preferably, the mRNA vaccine further comprises a lipid.

More preferably, the lipid includes, but is not limited to, a liposome capable of promoting self-assembly into virus-sized particles (appr. 100 nm), a liposome allowing intracellular release of mRNA from endosomes, a liposome supporting a phospholipid bilayer structure and a liposome working as a stabilizer.

More preferably, the lipid may further comprise a PEGylated lipid to increase the half-life of LNPs.

In one specific embodiment of the present invention, the lipid comprises a cationic lipid, a PEGylated lipid, cholesterol, and/or a phospholipid.

The mRNA vaccine disclosed herein can be a liposome, a lipid complex or a lipid nanoparticle. The liposome can be prepared in the following forms: 1,2-dioleyloxy-*N*,*N-*dimethylaminopropane (DODMA) liposome, 1,2-dilinoleyloxy-3-dimethylaminopropane (DLin-DMA) liposome or 2,2-dilinoleyl-4-(2-dimethylaminoethyl)-[1,3]-dioxolane (DLin-KC2-DMA) liposome. The lipid complex or lipid nanoparticle may be formed from a lipid selected from: DLin-DMA, DLin-K-DMA, 98N12-5, C12-200, DLin-MC3-DMA, DLin-KC2-DMA, DODMA, PLGA, PEG, PEG-DMG, PEGylated lipid and aminoalcohol lipid.

The mRNA vaccine disclosed herein may further comprise a pharmaceutically acceptable excipient. The pharmaceutically acceptable excipient may be a carrier, a diluent, an adjuvant or adjuvant-encoding nucleotide sequence, a solubilizer, a binder, a lubricant, a suspending agent, a transfection promoter or the like. Such transfection promoter includes, but is not limited to, surfactants such as immune stimulating complexes, Freunds incomplete adjuvant, LPS analogs (e.g., monophosphoryl lipid A), muramyl peptide, benzoquinone analogs, squalene, hyaluronic acid, lipids, liposomes, calcium ion, viral proteins, cations, polycations (e.g., poly-L-glutamic acid (LGS)), nanoparticles or other known transfection promoters. The nucleotide sequence of the coding adjuvant is a nucleotide sequence of at least one adjuvant as follows: GM-CSF, IL-17, IFNg, IL-15, IL-21, anti-PD1/2, lactoferrin, protamine, IL-1, IL-2, IL-3, IL-4, IL-5, IL-6, IL-7, IL-8, IL-9, IL-10, IL-12, INF-α, INF-γ, Lymphotoxin-α, hGH, MCP-1, MIP-1a, MIP-1p, IL-8, RANTES, L-selectin, P-selectin, E-selectin, CD34, GlyCAM-1, MadCAM-1, LFA-1, VLA-1, Mac-1, pl50.95, PECAM, ICAM-1, ICAM-2, ICAM-3, CD2, LFA-3, M-CSF, CD40, CD40L, vascular growth factor, fibroblast growth factor, nerve growth factor, vascular endothelial growth factor, Apo-1, p55, WSL-1, DR3, TRAMP, Apo-3, AIR, LARD, NGRF, DR4, DR5, KILLER, TRAII,-R2, TRICK2, DR6, caspase ICE, Fos, c-jun, Sp-1, Ap-1, Ap-2, p38, p65Rel, MyD88, IRAK, TRAF6, IkB, inactive NIK, SAP K, SAP-1, JNK, NFkB, Bax, TRAIL, TRAILrec, TRAILrecDRC5, TRAIL-R3, TRAIL-R4, RANK, RANK LIGAND, Ox40, Ox40LIGAND, NKG2D, MICA, MICB, NKG2A, NKG2B, NKG2C, NKG2E, NKG2F, TAP1, TAP2 and functional fragments thereof.

In a third aspect of the present invention, provided is a method for preparing an mRNA or mRNA composition, comprising mixing an mRNA with a cationic or polycationic compound, and packaging the mixture with a lipid.

Preferably, the lipid includes, but is not limited to, a liposome capable of promoting self-assembly into virus-sized particles (appr. 100 nm), a liposome allowing intracellular release of mRNA from endosomes, a liposome supporting a phospholipid bilayer structure and a liposome working as a stabilizer. More preferably, the lipid may further comprise a PEGylated lipid to increase the half-life of LNPs.

In one specific embodiment of the present invention, the lipid comprises a cationic lipid, a PEGylated lipid, cholesterol, and/or a phospholipid.

In a fourth aspect of the present invention, provided is a method for preparing an mRNA vaccine, comprising: mixing the mRNA or mRNA composition disclosed herein with a cationic or polycationic compound, and packaging the mixture with a lipid. Preferably, the mixture is packaged into lipid nanoparticles.

Preferably, the lipid includes, but is not limited to, a liposome capable of promoting self-assembly into virus-sized particles (appr. 100 nm), a liposome allowing intracellular release of mRNA from endosomes, a liposome supporting a phospholipid bilayer structure and a liposome working as a stabilizer. More preferably, the lipid may further comprise a PEGylated lipid to increase the half-life of LNPs.

In one specific embodiment of the present invention, the lipid comprises a cationic lipid, a PEGylated lipid, cholesterol, and/or a phospholipid.

In a fifth aspect of the present invention, provided is use of the mRNA or mRNA composition or the mRNA vaccine in preventing and/or treating a disease caused by SARS-CoV-2 infection. Preferably, the disease caused by SARS-CoV-2 infection includes, but is not limited to, COVID-19.

In a sixth aspect of the present invention, provided is use of the mRNA or mRNA composition or the mRNA vaccine in resisting SARS-CoV-2 infection.

In a seventh aspect of the present invention, provided is use of the mRNA or mRNA composition or the mRNA vaccine in preparing a medicament for preventing and/or treating a disease caused by SARS-CoV-2 infection.

Preferably, the disease caused by SARS-CoV-2 infection includes, but is not limited to, COVID-19.

In an eighth aspect of the present invention, provided is use of the mRNA or mRNA composition or the mRNA vaccine in preparing a medicament for resisting SARS-CoV-2 infection.

In a ninth aspect of the present invention, provided is a method for treating and/or preventing a disease caused by SARS-CoV-2 infection, comprising administering to an individual an effective amount of the mRNA or mRNA composition or the mRNA vaccine disclosed herein.

In a tenth aspect of the present invention, provided is a method for preventing SARS-CoV-2 infection, comprising administering to an individual not infected with SARS-CoV-2 an effective amount of the mRNA vaccine disclosed herein.

In an eleventh aspect of the present invention, provided is a method for treating SARS-CoV-2 infection, comprising administering to an individual infected with SARS-CoV-2 an effective amount of the mRNA or mRNA composition or the mRNA vaccine disclosed herein, such that the individual produces a neutralizing antibody against SARS-CoV-2.

In a twelfth aspect of the present invention, provided is a method for antibody screening, comprising administering to an individual an effective amount of the mRNA or mRNA composition or the mRNA vaccine disclosed herein.

The method for antibody screening is not a therapeutic method. The method is used for screening neutralizing antibodies, for detecting and comparing the efficacy of antibodies to determine which antibodies can be used as drugs and which cannot, or for comparing the sensitivity to efficacies of various drugs, i.e., the therapeutic effect is not necessary but only possible.

In a thirteenth aspect of the present invention, provided is a method for inducing a neutralizing antigen-specific immune response in an individual, comprising administering to the individual the mRNA or mRNA composition or the mRNA vaccine disclosed herein.

Preferably, the antigen-specific immune response comprises a T cell response and/or a B cell response.

In a fourteenth aspect of the present invention, provided is a protein encoded by the mRNA or mRNA composition disclosed herein.

Preferably, the protein is the full-length S protein fixed in the pre-fusion conformation. More preferably, the full-length S protein fixed in the pre-fusion conformation comprises a mutation at positions 682RRAR685 and/or a mutation at positions 986KV987, such that the S protein is fixed in the pre-fusion conformation. Most preferably, the full-length S protein fixed in the pre-fusion conformation is obtained by mutating 682RRAR685 of the wild-type full-length S protein to GSAG and/or 986KV987 to PP.

In a fifteenth aspect of the present invention, provided is a nucleotide sequence encoding the protein disclosed herein.

In a sixteenth aspect of the present invention, provided is a vector comprising the nucleotide sequence disclosed herein.

In a seventeenth aspect of the present invention, provided is a cell comprising the protein, the nucleotide sequence and/or the vector disclosed herein.

The mRNA or mRNA composition and the mRNA vaccine comprising the mRNA or mRNA composition disclosed herein have the advantages that: 1, the present invention is synthesized *in vitro*, without cell culture process and the risk of animal source material contamination; 2, the present invention can be rapidly developed and produced, and is suitable for standardized production and easy for mass production and quality control, and the same production flow is applicable to a plurality of different products; 3, the present invention can be expressed continuously in a period of time, providing prolonged antigen exposure time and improved strength and quality of immune response; 4, the present invention simulates the process of natural infection, and the protein is translated and modified in human cells, can be presented by MHC class I molecules, and thus induces stronger cellular immunity; 5, the present invention supports various protein forms including intracellular proteins, transmembrane proteins, VLPs and the like, and can avoid the purification problems due to the low yield of VLP; 6, the present invention has self-adjuvant effect, and adjuvant screening is not required; 7, the present invention has no risk of infection and genome integration; 8, in case of no pre-existing immunity, the present invention can be used for multiple immunizations.

The expression product of the mRNA or mRNA composition disclosed herein comprises the RBD or the variant thereof and the S protein or the variant thereof, wherein the RBD comprises a major neutralizing epitope which can induce a high titer of neutralizing antibodies; moreover, less non-neutralizing epitopes are present, resulting in higher safety. The full-length S protein can induce a high level of specific cellular immunity, and can generate an extremely excellent immune effect in combination with an RBD capable of specifically inducing a neutralizing antibody. Furthermore, the examples also demonstrate that the expression products of the mRNA or mRNA composition disclosed herein are capable of inducing a high level of neutralizing antibodies and cytokines in humans.

Also, some of the disclosures in the prior art also support the technical scheme of the present invention. For example, by sequence analysis and structure modeling prediction of the RBD of SARS-CoV-2, it was reported that the S protein of SARS-CoV-2 maintains the structural conformation of the interaction of the S protein of SARS-CoV and ACE2 (see Xintian, X et al. (2020), "Evolution of the novel coronavirus from the ongoing Wuhan outbreak and modeling of its spike protein for risk of human transmission", Sci China Life Sci.). By comparing different fragments of the RBD of MERS-CoV, protein fragment 377-588 was determined as the key neutralizing domain, i.e., the domain eliciting the highest neutralizing antibody titer in mouse and rabbit models; also, this key neutralizing domain can still bind to the receptor hDPP4, demonstrating that it maintains the structural conformation, and can provide linear epitopes as well as structural epitopes (see Cuiqing, M et al. (2014), "Searching for an ideal vaccine candidate among different MERS coronavirus receptor-binding fragments--the importance of immunofocusing in subunit vaccine design", Vaccine. 32(46):6170-6176).

The term "individual" used herein includes mammals and humans. Such mammals include, but are not limited to, rodents (e.g., mice and rats), monkeys, zebrafish, pigs, chickens, rabbits, and the like.

The term "prevention" used herein refers to all actions to avoid a symptom or to delay a particular symptom by administering a product as described herein before or after onset of a disease; preferably, the prevention comprises using the mRNA or mRNA composition disclosed herein as a vaccine.

The term "treatment" used herein refers to therapeutic intervention to ameliorate the signs, symptoms, etc. of a disease or pathological condition after onset of the disease; preferably, the treatment comprises screening for antibodies that bind to the mRNA or mRNA composition disclosed herein and using the antibodies for treatment.

The term "effective amount" used herein refers to an amount or dose of a product described herein which provides desired therapeutic or prophylactic effect after administration to a patient or an organ in a single or multiple doses.

The "S protein" is a structural protein constituting SARS-CoV-2, and is known as spike protein. The "RBD" is a structural protein constituting SARS-CoV-2 and is known as spike protein receptor binding domain.

The term "identity" used herein refers to that in the context of using an amino acid sequence or a nucleotide sequence, one skilled in the art can adjust the sequence as necessary for practical work to obtain a sequence having (including but not limited to) 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8% or 99.9% similarity and still maintaining identical or similar functions as the original amino acid sequence or nucleotide sequence. For example, an amino acid sequence having identity to the original sequence still keeps the function of inducing neutralizing antibodies and producing cytokines *in vivo*, and the expression product of an mRNA sequence having identity to the original sequence still keeps the function of inducing neutralizing antibodies and producing cytokines *in vivo.*

### BRIEF DESCRIPTION OF THE DRAWINGS

Hereinafter, embodiments of the present invention will be described in detail with reference to the accompanying drawings, in which:
FIG. 1: sequencing result of the sequence encoding the wild-type SARS-CoV-2 RBD with tPA signal peptide.
FIG. 2: sequencing result of the sequence encoding the wild-type SARS-CoV-2 S protein, combining FIG. 2A and FIG. 2B.
FIG. 3: sequence map of a basic plasmid template comprising T7 promoter, 5' UTR, 3' UTR and poly A tail.
FIG. 4: detection of capped mRNA in denaturing formaldehyde gel after purification, wherein M denotes marker, 1 denotes the mRNA encoding the wild-type SARS-CoV-2 RBD with tPA signal peptide, and 2 denotes the mRNA encoding the full-length S protein fixed in the pre-fusion conformation with a mutation to GSAG at 682RRAR685 and a mutation to PP at 986KV987.
FIG. 5: WB (western blot) results, wherein 1 denotes the mRNA encoding the wild-type SARS-CoV-2 RBD with tPA signal peptide, and 2 denotes the negative control.
FIG. 6: immunofluorescence result.
FIG. 7: particle size and the particle size distribution result of mRNA-LNP by DLS (dynamic light scattering), wherein A denotes RBD+S-1-LNP, B denotes RBD+S-2-LNP, C denotes RBD+S-3-LNP, D denotes RBD-LNP, and E denotes S-LNP.
FIG. 8: mRNA integrity result of the packaged sample by formaldehyde denatured gel, wherein 1 denotes the mRNA encoding the wild-type SARS-CoV-2 RBD with tPA signal peptide, 2 denotes RBD+S-1, 3 denotes RBD+S-2, 4 denotes RBD+S-3, and 5 denotes the mRNA encoding the full-length S protein fixed in the pre-fusion conformation with a mutation to GSAG at 682RRAR685 and a mutation to PP at 986KV987.
FIG. 9: S protein-specific antibody titer after primary and secondary immunizations, wherein Negative denotes the negative control.
FIG. 10: interferon gamma (IFN-γ) detection by ELISpot (enzyme linked immunospot), wherein the ordinate denotes spot forming unit (SFU) per million splenocytes, and Negative denotes the negative control.
FIG. 11: IFN-γ, interleukin-2 (IL-2) and tumor necrosis factor-alpha (TNF-α) detections by CD4 CK intracellular staining (ICS).
FIG. 12: IFN-γ, interleukin-2 (IL-2) and tumor necrosis factor-alpha (TNF-α) detections by CD8 CK intracellular staining (ICS).

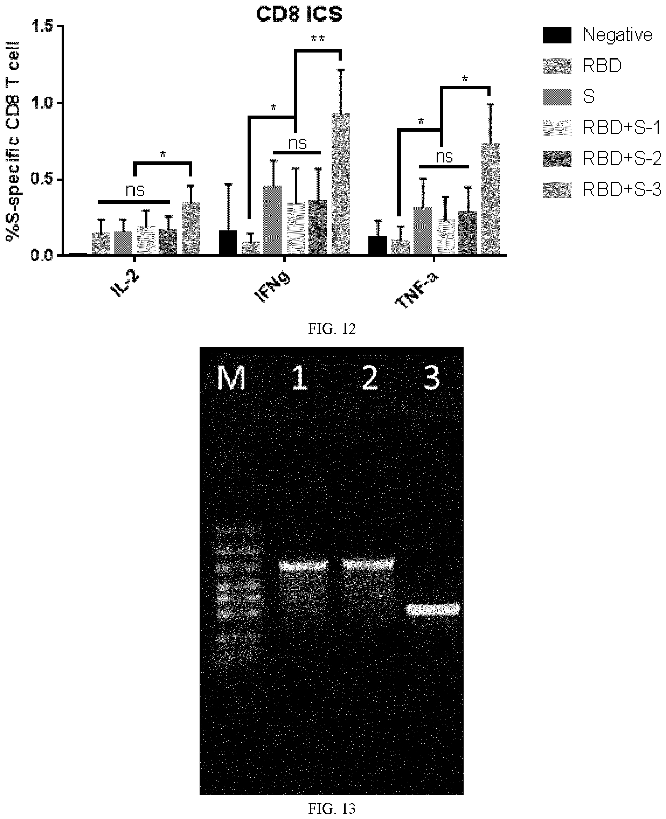

FIG. 13: detection of capped mRNA in denaturing formaldehyde gel after purification, wherein M denotes marker, 1 denotes the mRNA encoding the full-length S protein fixed in the pre-fusion conformation with a mutation to GSAG at 682RRAR685 and a mutation to PP at 986KV987 prepared in Example 1, 2 denotes the mRNA encoding the full-length S protein of 501Y.V2 strain fixed in the pre-fusion conformation with a mutation to GSAG at 682RRAR685 and a mutation to PP at 986KV987, and 3 denotes the mRNA encoding the wild-type SARS-CoV-2 RBD sequence of 501Y.V2 strain with IgE signal peptide. The results suggested that the mRNA was of the correct size and was essentially free of degradation.

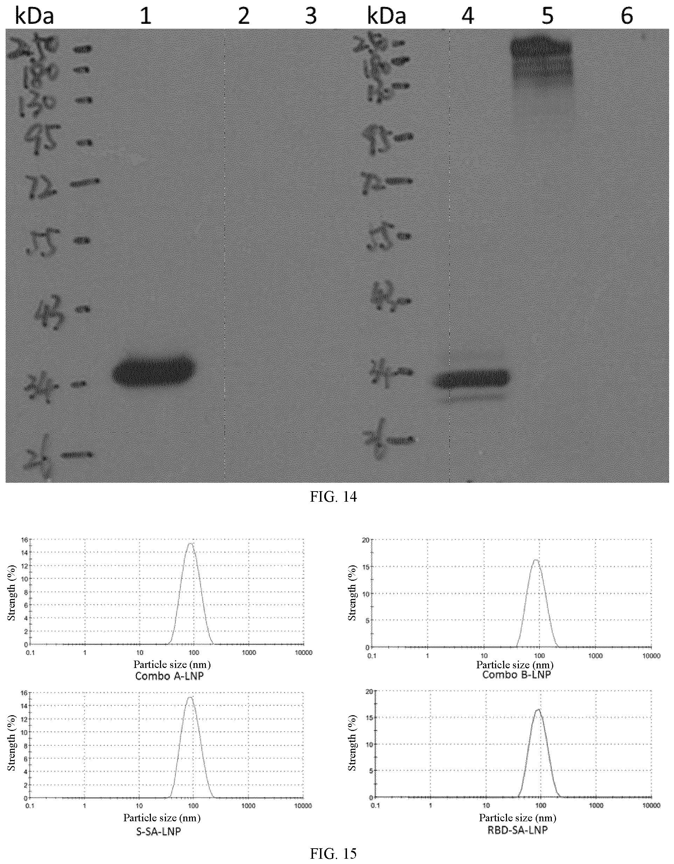

FIG. 14: WB assay results, wherein 1 denotes the expression supernatant of the mRNA encoding the wild-type SARS-CoV-2 RBD of 501Y.V2 strain with IgE signal peptide, 2 denotes the expression supernatant of the mRNA encoding the full-length S protein of 501Y.V2 strain fixed in the pre-fusion conformation with a mutation to GSAG at 682RRAR685 and a mutation to PP at 986KV987, 3 denotes the cell supernatant of the negative control, 4 denotes the expression cell pellet of the mRNA encoding the wild-type SARS-CoV-2 RBD sequence of 501Y.V2 strain with IgE signal peptide, 5 denotes the expression cell pellet of the mRNA encoding the full-length S protein of 501Y.V2 strain fixed in the pre-fusion conformation with a mutation to GSAG at 682RRAR685 and a mutation to PP at 986KV987, and 6 denotes the cell pellet of the negative control.
FIG. 15: particle size and the particle size distribution result of mRNA-LNP by DLS (dynamic light scattering).
FIG. 16: titer of S protein-specific antibody for 501Y.V2 strain after primary and secondary immunizations.
FIG. 17: titer of S protein-specific antibody for Wuhan-Hu-1 isolate after primary and secondary immunizations.
FIG. 18: alternative neutralizing antibody titers, wherein the 4 groups on the left are the alternative neutralizing antibody titer against Wuhan-Hu-1 isolate and the 4 group on the right are the alternative neutralizing antibody titer against the 501Y.V2 strain.
FIG. 19: cellular immune response detection in CD4+ T cell Th1 subtype.
FIG. 20: cellular immune response detection in CD8+ T cell Th1 subtype.

### DETAILED DESCRIPTION

Technical schemes in the examples of the present invention will be described clearly and completely in conjunction with the accompanying drawings. It is apparent that the examples described herein are only some examples of the present invention, but not all of them. Based on the examples of the present invention, all other examples obtained by those of ordinary skill in the art without creative work shall fall within the protection scope of the present invention. Sources of reagents used in the examples:
Neutralizing antibodies in the serum of immunized mice were detected by alternative neutralizing antibody assay through competitive binding of ACE2 protein and RBD protein.

Specific antibodies in the serum of immunized mice were detected by specific antibody detection using RBD protein.

The RBD protein used for Wuhan-Hu-1 isolate was a wild-type RBD protein (manufacturer: Genscript, Cat. No.: Z03483-1).

The RBD protein used for 501Y.V2 strain was an RBD protein with 501Y.V2 strain mutations (manufacturer: Novoprotein, Cat. No.: DRA125).

### Example 1: Preparation and detection of mRNA

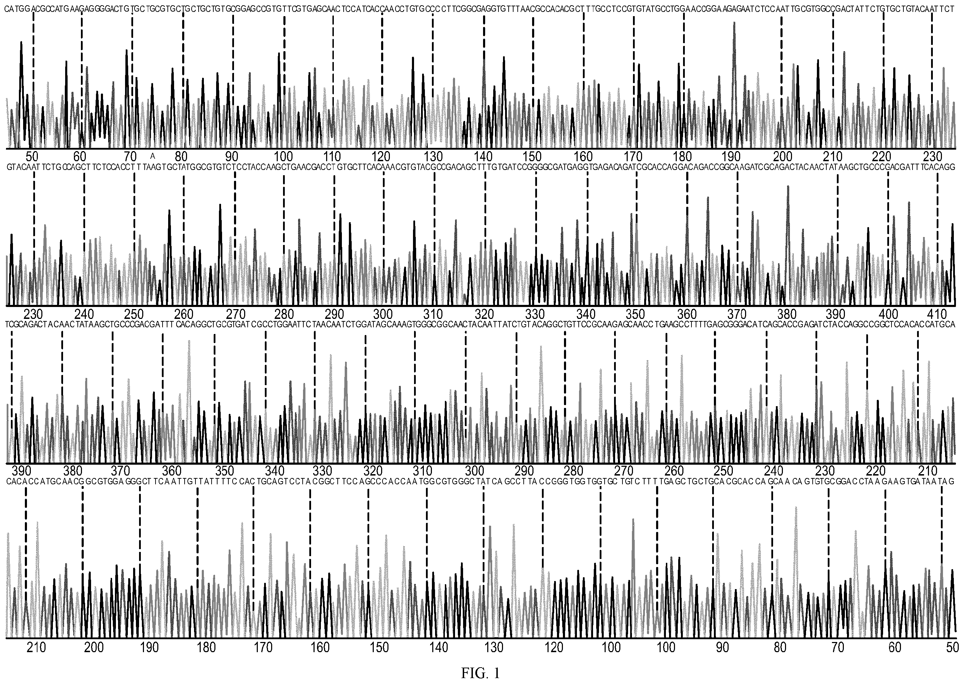

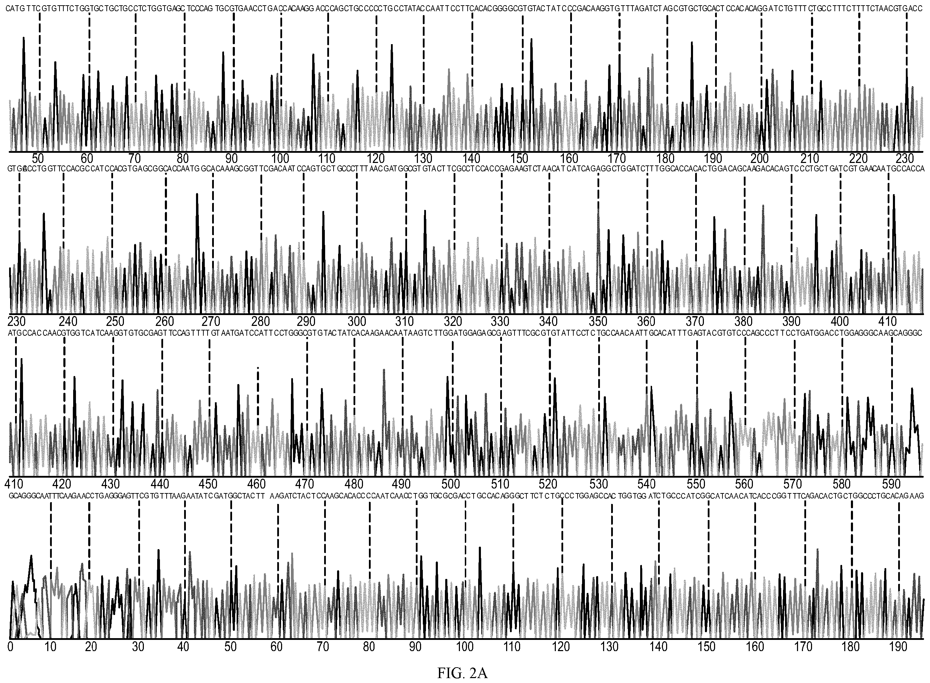

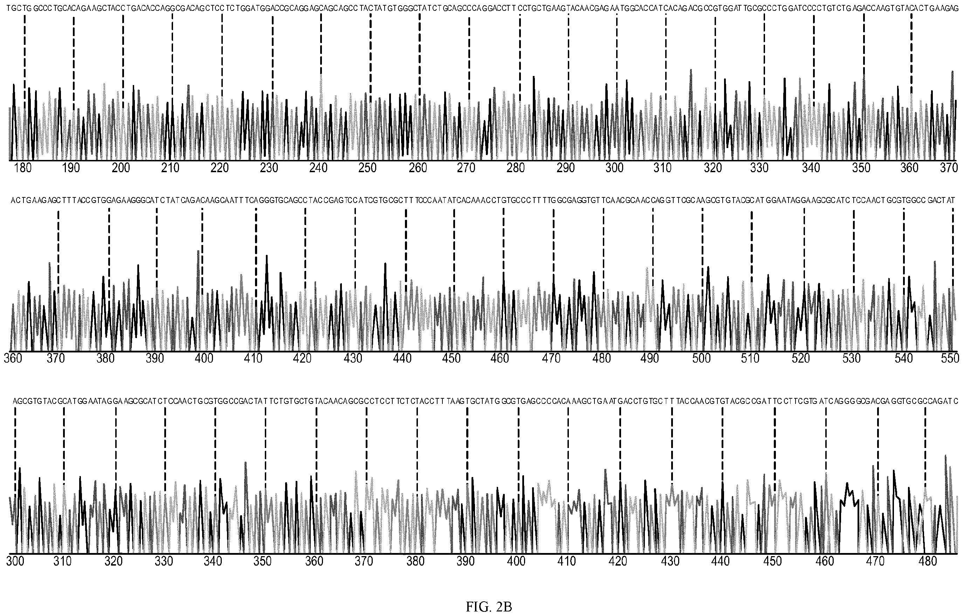

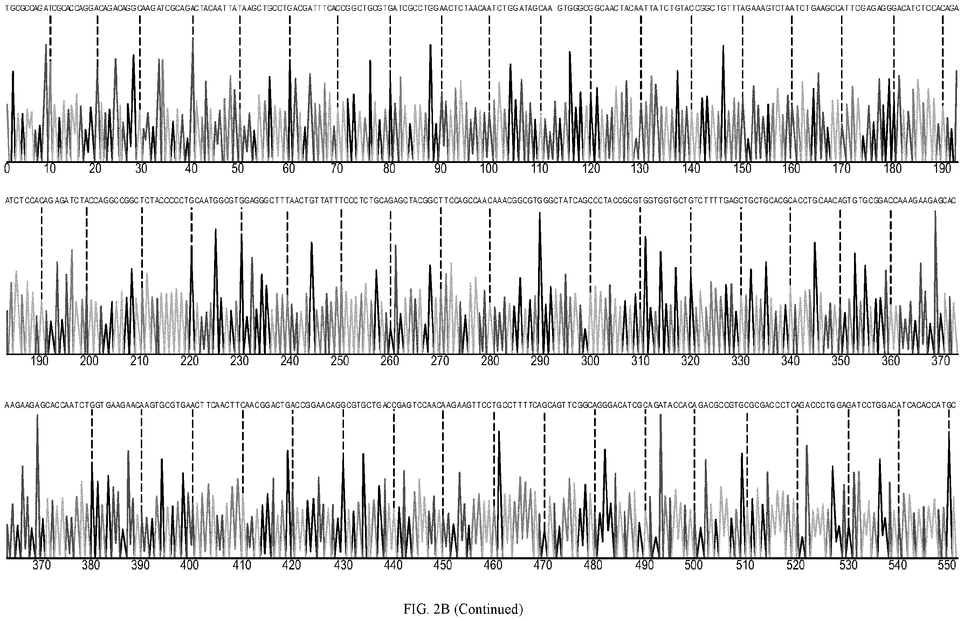

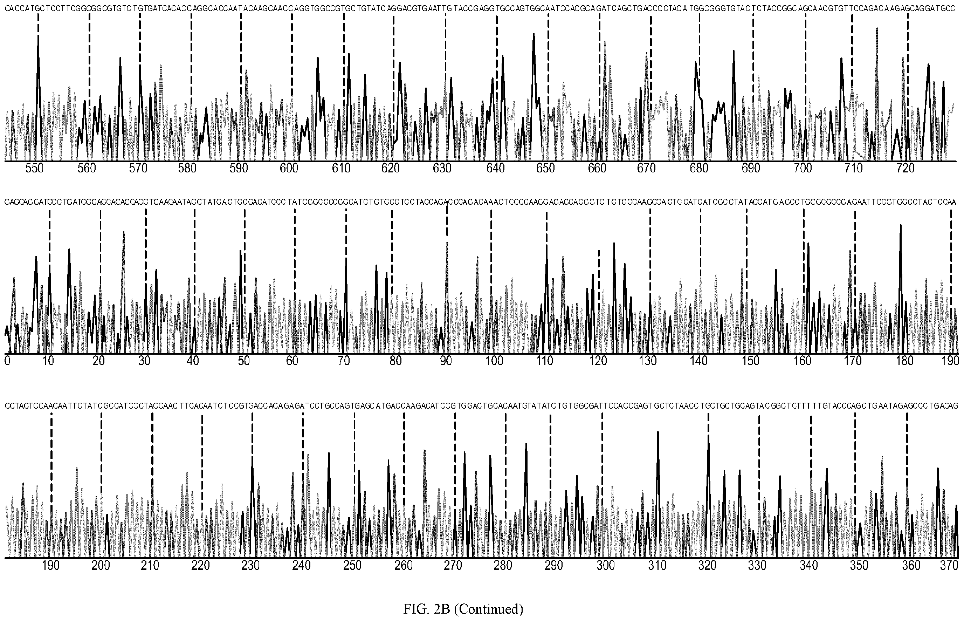

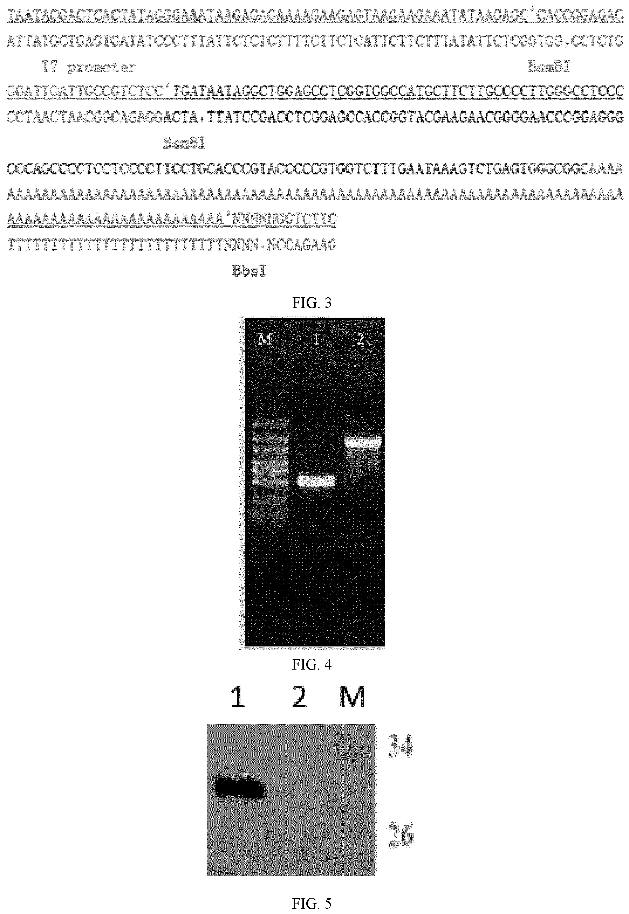

1. Designed gene sequences of the antigens were artificially synthesized.
2. Short nucleotide chains (primers) were synthesized through the solid phase phosphoramidite triester method.
3. The primers were mutually used as templates for PCR amplification.
4. The amplification product in step 3 was ligated into pUC57 vector, transformed and sequenced.
5. The sequence was confirmed consistent as expected by sequencing, and the results are shown in FIGs. 1-2. Specifically, FIG. 1 shows the sequencing result of the sequence encoding the wild-type SARS-CoV-2 RBD with tPA signal peptide, with the nucleotide sequence set forth in SEQ ID NO: 4 and the amino acid sequence set forth in SEQ ID NO: 3. FIG. 2 shows the sequencing results of the sequences encoding the full-length S protein fixed in the pre-fusion conformation with a mutation to GSAG at 682RRAR685 and a mutation to PP at 986KV987, with the nucleotide sequence set forth in SEQ ID NO: 5 and the amino acid sequence set forth in SEQ ID NO: 2.
6. A base plasmid template comprising T7 promoter, 5' UTR, 3' UTR and polyA tail were prepared, with the sequence set forth in FIG. 3 (SEQ ID NO: 8).
7. The template was subjected to PCR with homologous primers, and the result suggested a correct outcome.

The basic plasmid template was linearized with restriction endonuclease BsmBI. The PCR products were ligated to basic plasmid templates by homologous recombination, and were used for transforming an Xl1-Blue strain. The sequencing result confirmed that the sequence was correct and the transcription template was successfully constructed. The strain was cultured in shake flasks, and purified by a large-extraction kit free of endotoxin to obtain a transcription template.

The transcription template was linearized using restriction endonuclease BbsI. A T7 *in-vitro* transcription kit was used for transcription to obtain uncapped mRNA sequences set forth in SEQ ID NOs: 4-5 (the specific mRNA sequences are set forth in SEQ ID NOs: 16-17, respectively). The transcription templates were digested with DNaseI, and the mRNA was purified by precipitation. mRNA was capped with Cap1 capping kit and the capped mRNA was purified with mRNA purification kit. The purified mRNA was dissolved in an acidic sodium citrate buffer for later use.

The capped mRNA was detected in denaturing formaldehyde gel after purification, as shown in FIG. 4, wherein M denotes marker, 1 denotes the mRNA encoding the wild-type SARS-CoV-2 RBD with tPA signal peptide, and 2 denotes the mRNA encoding the full-length S protein fixed in the pre-fusion conformation with a mutation to GSAG at 682RRAR685 and a mutation to PP at 986KV987. The results suggested that the mRNA was of the correct size and was essentially free of degradation.

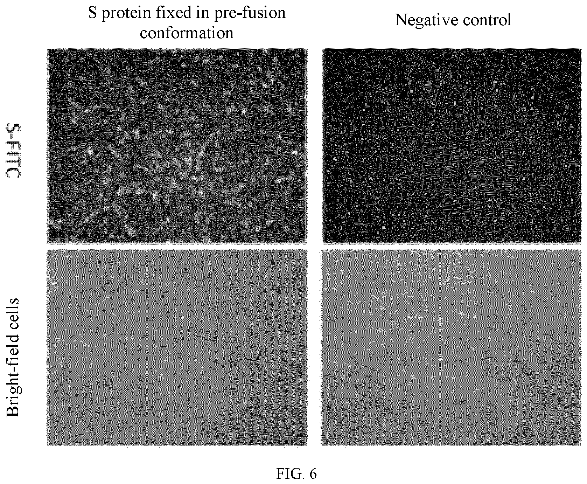

HEK293 cells were transferred into 3 wells on a 24-well plate. Cells in the wells 1 and 2 were transfected using lipofectamine 2000 transfection agent with 0.5 µg of the capped and purified mRNA encoding the wild-type SARS-CoV-2 RBD with tPA signal peptide and the mRNA encoding the full-length S protein fixed in the pre-fusion conformation with a mutation to GSAG at 682RRAR685 and a mutation to PP at 986KV987, respectively, and lipofectamine 2000 transfection agent was added in well 3 as the negative control. After 24 h of transfection, cell supernatants of wells 1 and 3 were subjected to WB assay, and cells of wells 2 and 3 were fixed and subjected to immunofluorescent assay with anti-S protein polyclonal antibody. The WB assay results are shown in FIG. 5, wherein 1 denotes the mRNA encoding the wild-type SARS-CoV-2 RBD with tPA signal peptide, and 2 denotes the negative control. The results showed that the expressed protein was of correct size. The immunofluorescent assay results are shown in FIG. 6, demonstrating that the mRNA encoding the full-length S protein fixed in the pre-fusion conformation with a mutation to GSAG at 682RRAR685 and a mutation to PP at 986KV987 can be normally expressed.

### Example 2: Preparation of mRNA vaccines and immunization

### 1. Material preparation

1) Cationic lipid D-Lin-MC3-DMA, distearoyl phosphatidylcholine (DSPC), cholesterol, and PEGylated lipid PEG-DMG were dissolved and mixed in ethanol in a molar ratio of 50:10:38.5:1.5.
2) The mRNA encoding the wild-type SARS-CoV-2 RBD with tPA signal peptide and the mRNA encoding the full-length S protein fixed in the pre-fusion conformation with a mutation to GSAG at 682RRAR685 and a mutation to PP at 986KV987 prepared in Example 1 were mixed in mass ratios of 1:1, 2:1, 1:2 to obtain mRNA mixtures, respectively marked as RBD+S-1, RBD+S-2 and RBD+S-3.

### 2. Procedures

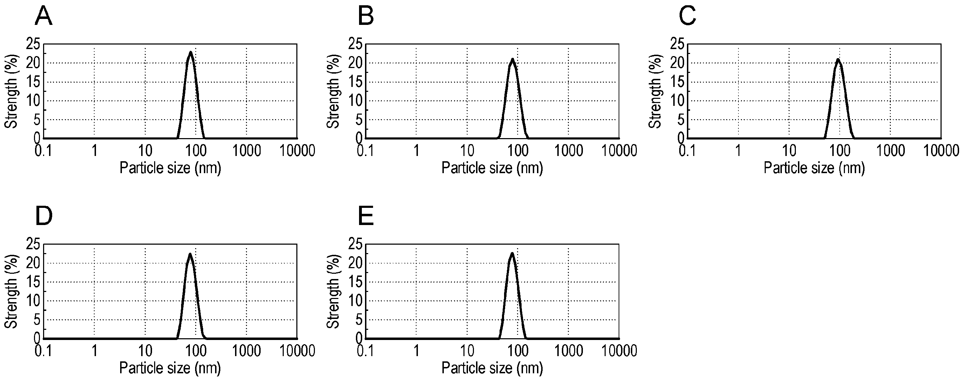

RBD+S-1, RBD+S-2, RBD+S-3, the mRNA encoding the wild-type SARS-CoV-2 RBD with tPA signal peptide and the mRNA encoding the full-length S protein fixed in the pre-fusion conformation with a mutation to GSAG at 682RRAR685 and a mutation to PP at 986KV987 were packaged in Precision Nanosystems Ignite instrument in a flow ratio of lipid mixture:mRNA = 1:3. The packaged mRNA-LNP (LNP refers to lipid nanoparticle) was dialyzed into DPBS, ultrafiltered and concentrated, and a sample for subsequent animal studies was obtained after sterile filtration. The particle size and the particle size distribution of mRNA-LNP were detected by DLS, and the detection result is shown in FIG. 7. The particle sizes of the packaged samples were 70-100 nm, and the PDI was less than 0.2. Among these, RBD+S-1-LNP had an average particle size of 77.15 nm, a PDI of 0.038 and an intercept of 0.958, as shown in Table 1; RBD+S-2-LNP had an average particle size of 77.04 nm, a PDI of 0.055 and an intercept of 0.959, as shown in Table 2; RBD+S-3-LNP had an average particle size of 91.43 nm, a PDI of 0.049 and an intercept of 0.974, as shown in Table 3; RBD-LNP had an average particle size of 77.92 nm, a PDI of 0.036 and an intercept of 0.954, as shown in Table 4; S-LNP had an average particle size of 76.89 nm, a PDI of 0.031 and an intercept of 0.977, as shown in Table 5.

**Table 1: RBD+S-1-LNP**

| | Particle size (nm) | Strength (%) | Standard deviation |
|---|---|---|---|
| Peak 1 | 81.06 | 100 | 18.64 |
| Peak 2 | 0 | 0 | 0 |
| Peak 3 | 0 | 0 | 0 |

**Table 2: RBD+S-2-LNP**

| | Particle size (nm) | Strength (%) | Standard deviation |
|---|---|---|---|
| Peak 1 | 81.93 | 100 | 20.68 |
| Peak 2 | 0 | 0 | 0 |
| Peak 3 | 0 | 0 | 0 |

**Table 3: RBD+S-3-LNP**

| | Particle size (nm) | Strength (%) | Standard deviation |
|---|---|---|---|
| Peak 1 | 97 | 100 | 24.54 |
| Peak 2 | 0 | 0 | 0 |
| Peak 3 | 0 | 0 | 0 |

**Table 4: RBD-LNP**

| | Particle size (nm) | Strength (%) | Standard deviation |
|---|---|---|---|
| Peak 1 | 82.01 | 100 | 19.41 |
| Peak 2 | 0 | 0 | 0 |
| Peak 3 | 0 | 0 | 0 |

**Table 5: S-LNP**

| | Particle size (nm) | Strength (%) | Standard deviation |
|---|---|---|---|
| Peak 1 | 80.72 | 100 | 18.76 |
| Peak 2 | 0 | 0 | 0 |
| Peak 3 | 0 | 0 | 0 |

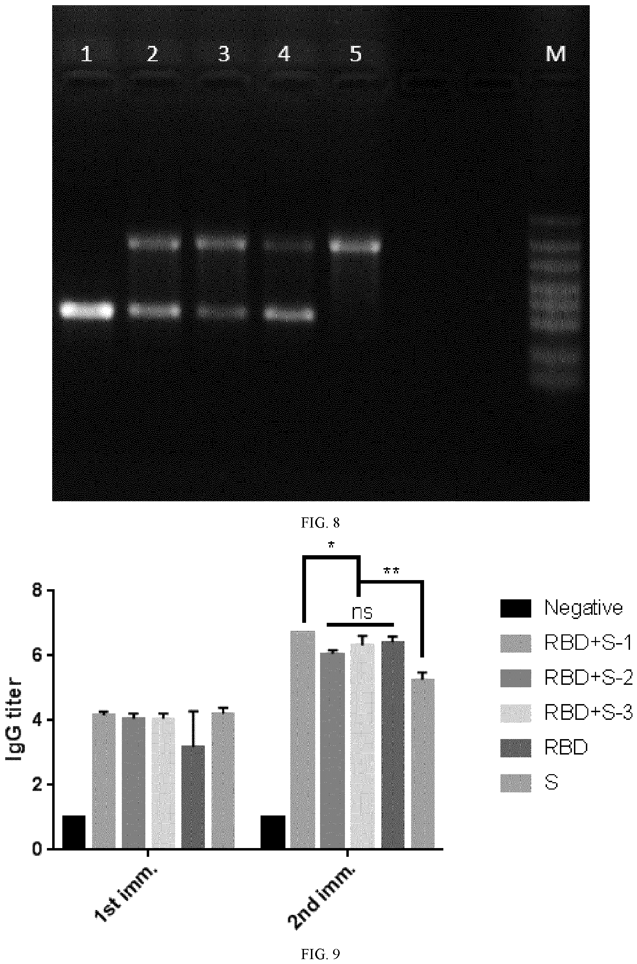

mRNA integrity of the packaged sample was detected in formaldehyde denatured gel, as shown in FIG. 8, wherein 1 denotes the mRNA encoding the wild-type SARS-CoV-2 RBD with tPA signal peptide, 2 denotes RBD + S-1, 3 denotes RBD + S-2, 4 denotes RBD + S-3, and 5 denotes the mRNA encoding the full-length S protein fixed in the pre-fusion conformation with a mutation to GSAG at 682RRAR685 and a mutation to PP at 986KV987. The results suggested that the mRNA was essentially free of degradation.

BALB/c female mice aged about 6 weeks were randomized into 6 groups each containing 6 mice. The mice were intramuscularly administered at 10 µg on days 0 and 28. S protein-specific antibody titer was measured on days 28 and 42. On day 42, the mice were sacrificed and the cytokine was measured.

### 3. Results

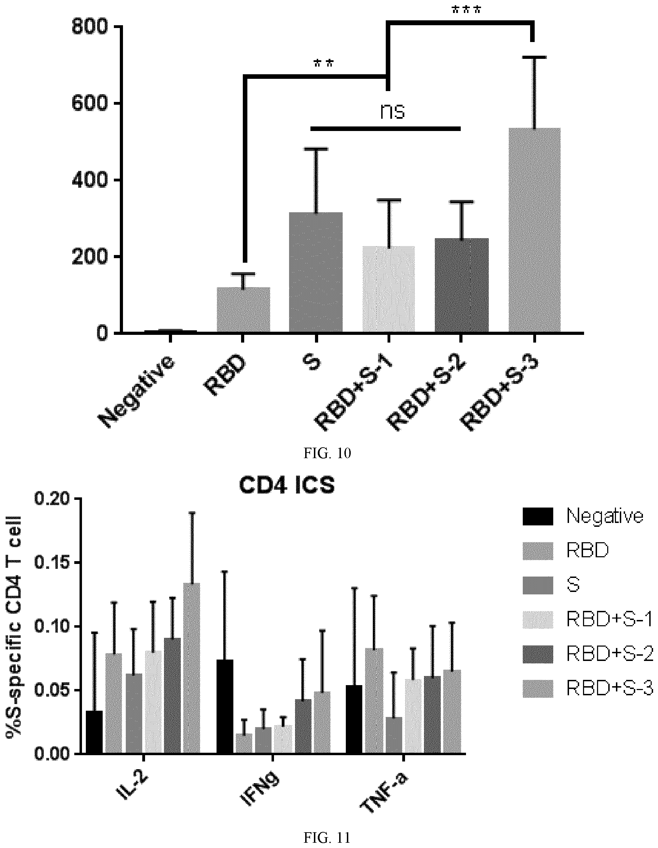

The S protein-specific antibody titers after the primary and secondary immunizations are shown in FIG. 9. It can be seen that after the secondary immunization, the specific antibody titer of full-length S protein immunization alone was significantly lower than that of RBD immunization alone; in the three groups receiving combined S+RBD immunization, two groups had no significant difference from RBD immunization alone in the specific antibody titer, and only the RBD+S-1 group showed a synergistic effect and a specific antibody titer significantly higher than that of RBD immunization alone. The results of interferon gamma (IFN-γ) detection by ELISpot are shown in FIG. 10. It can be seen that the IFN-γ secretion of full-length S protein immunization alone was significantly higher than that of RBD immunization alone; in the three groups receiving combined S+RBD immunization, two groups had no significant difference from full-length S protein immunization alone in IFN-γ secretion, and only RBD+S-3 showed a synergistic effect and an IFN-γ secretion level significantly higher than that of full-length S protein immunization alone. IFN-γ, interleukin-2 (IL-2) and tumor necrosis factor-alpha (TNF-α) detection results by CK intracellular staining (ICS) are shown in FIGs. 11 and 12. CD4+ T cells showed low response and great individual difference, thus suggesting less significance; the result of CD8+ T cells was basically consistent with that of ELISpot. In the three groups receiving combined S+RBD immunization, two groups had no significant difference from full-length S protein immunization alone in secretions of IFN-γ, IL-2 and TNF-α, and only RBD+S-3 showed significant synergistic effect. The results suggested that the combination of the full-length S protein and the RBD can integrate the cellular immune advantages of the full-length S protein and the humoral immune advantages of the RBD, achieving synergistic effect of gain and superior effect in the prevention of 2019-nCoV infection.

### Example 3: Preparation and detection of mRNA with sequence derived from different SARS-CoV-2 mutants

1. The sequence of the wild-type SARS-CoV-2 RBD with IgE signal peptide was synthesized by amplification with primers used as templates of each other, wherein the RBD sequence contained K417N, E484K and N501Y mutations of 501Y.V2 strain, with the nucleotide sequence set forth in SEQ ID NO: 12 and the amino acid sequence set forth in SEQ ID NO: 13.
2. The sequence encoding the full-length S protein of 501Y.V2 strain fixed in the pre-fusion conformation with a mutation to GSAG at 682RRAR685 and a mutation to PP at 986KV987 was synthesized by amplification with primers used as templates of each other, wherein the sequences contained L18F, D80A, D215G, L242-L244 deletions (L242-244del), R246I, K417N, E484K, N501Y and A701V of 501Y.V2 strain, with the nucleotide sequence set forth in SEQ ID NO: 14 and the amino acid sequence set forth in SEQ ID NO: 15.
3. A base plasmid template comprising T7 promoter, 5' UTR, 3' UTR and polyA tail were prepared, with the sequence set forth in FIG. 3 (SEQ ID NO: 8).
4. The template was subjected to PCR with homologous primers, and the result suggested a correct outcome.

The basic plasmid template was linearized with restriction endonuclease BsmBI. The PCR products were ligated to basic plasmid templates by homologous recombination, and were used for transforming an Xl1-Blue strain. The sequencing result confirmed that the sequence was correct and the transcription template was successfully constructed. The strain was cultured in shake flasks, and purified by a large-extraction kit free of endotoxin to obtain a transcription template.

The transcription template was linearized using restriction endonuclease BbsI. A T7 *in-vitro* transcription kit was used for transcription to obtain uncapped mRNA sequences set forth in SEQ ID NOs: 12 and 14 (the specific mRNA sequences are set forth in SEQ ID NOs: 18 and 19, respectively). The transcription templates were digested with DNaseI, and the mRNA was purified by precipitation. mRNA was capped with Cap1 capping kit and the capped mRNA was purified with mRNA purification kit. The purified mRNA was dissolved in an acidic sodium citrate buffer for later use.

The capped mRNA was detected in denaturing formaldehyde gel after purification, as shown in FIG. 13, wherein M denotes marker, 1 denotes the mRNA encoding the full-length S protein fixed in the pre-fusion conformation with a mutation to GSAG at 682RRAR685 and a mutation to PP at 986KV987 prepared in Example 1, 2 denotes the mRNA encoding the full-length S protein of 501Y.V2 strain fixed in the pre-fusion conformation with a mutation to GSAG at 682RRAR685 and a mutation to PP at 986KV987, and 3 denotes the mRNA encoding the wild-type SARS-CoV-2 RBD sequence of 501Y.V2 strain with IgE signal peptide. The results suggested that the mRNA was of the correct size and was essentially free of degradation. HEK293 cells were transferred into 3 wells on a 24-well plate. Cells in the wells 1 and 2 were transfected using lipofectamine 2000 transfection agent with 0.5 µg of the capped and purified mRNA encoding the wild-type SARS-CoV-2 RBD of 501Y.V2 strain with IgE signal peptide and the mRNA encoding the full-length S protein of 501Y.V2 strain fixed in the pre-fusion conformation with a mutation to GSAG at 682RRAR685 and a mutation to PP at 986KV987, respectively, and cells in well 3 were the negative control. After 24 h of transfection, the cell supernatant and cell precipitate were separated by centrifugation and subjected to WB assay. The WB assay results are shown in FIG. 14, wherein 1 denotes the expression supernatant of the mRNA encoding the wild-type SARS-CoV-2 RBD of 501Y.V2 strain with IgE signal peptide, 2 denotes the expression supernatant of the mRNA encoding the full-length S protein of 501Y.V2 strain fixed in the pre-fusion conformation with a mutation to GSAG at 682RRAR685 and a mutation to PP at 986KV987, 3 denotes the cell supernatant of the negative control, 4 denotes the expression cell pellet of the mRNA encoding the wild-type SARS-CoV-2 RBD sequence of 501Y.V2 strain with IgE signal peptide, 5 denotes the expression cell pellet of the mRNA encoding the full-length S protein of 501Y.V2 strain fixed in the pre-fusion conformation with a mutation to GSAG at 682RRAR685 and a mutation to PP at 986KV987, and 6 denotes the cell pellet of the negative control. The results showed that the expressed protein was of correct size.

### Example 4: Preparation of combined mRNA vaccines with sequence derived from different SARS-CoV-2 mutants and immunization

### 1. Material preparation

1) Cationic lipid D-Lin-MC3-DMA, distearoyl phosphatidylcholine (DSPC), cholesterol, and PEGylated lipid PEG-DMG were dissolved and mixed in ethanol in a molar ratio of 50:10:38.5:1.5.
2) The mRNA encoding the full-length S protein fixed in the pre-fusion conformation with a mutation to GSAG at 682RRAR685 and a mutation to PP at 986KV987 prepared in Example 1, the mRNA encoding the full-length S protein of 501Y.V2 strain fixed in the pre-fusion conformation with a mutation to GSAG at 682RRAR685 and a mutation to PP at 986KV987 prepared in Example 3, and the mRNA encoding the wild-type SARS-CoV-2 RBD sequence of 501Y.V2 strain with IgE signal peptide prepared in Example 3 were prepared.
3) The mRNA encoding the full-length S protein of 501Y.V2 strain fixed in the pre-fusion conformation with a mutation to GSAG at 682RRAR685 and a mutation to PP at 986KV987 prepared in Example 3 and the mRNA encoding the wild-type SARS-CoV-2 RBD sequence of 501Y.V2 strain with IgE signal peptide prepared in Example 3 were mixed in a mass ratio of 1:2 to obtain an mRNA mixture, abbreviated as combo A.
4) The mRNA encoding the full-length S protein fixed in the pre-fusion conformation with a mutation to GSAG at 682RRAR685 and a mutation to PP at 986KV987 prepared in Example 1 and the mRNA encoding the wild-type SARS-CoV-2 RBD sequence of 501Y.V2 strain with IgE signal peptide prepared in Example 3 were mixed in a mass ratio of 1:2 to obtain an mRNA mixture, abbreviated as combo B.

### 2. Procedures

Combo A, combo B, the mRNA encoding the full-length S protein of 501Y.V2 strain fixed in the pre-fusion conformation with a mutation to GSAG at 682RRAR685 and a mutation to PP at 986KV987 prepared in Example 3 and the mRNA encoding the wild-type SARS-CoV-2 RBD sequence of 501Y.V2 strain with IgE signal peptide prepared in Example 3 were packaged in Precision Nanosystems Ignite instrument in a flow ratio of lipid mixture:mRNA = 1:3, abbreviated as combo A-LNP, combo B-LNP, S-SA-LNP and RBD-SA-LNP, respectively. The packaged mRNA-LNP was dialyzed into DPBS, ultrafiltered and concentrated, and a sample for subsequent animal studies was obtained after sterile filtration. The particle size and the particle size distribution of mRNA-LNP were detected by DLS, and the detection result is shown in FIG. 15. The particle sizes of the packaged samples were 70-100 nm, and the PDI was less than 0.2. Among these, combo A-LNP had an average particle size of 79.87 nm, a PDI of 0.132 and an intercept of 0.962, as shown in Table 6; combo B-LNP had an average particle size of 80.61 nm, a PDI of 0.123 and an intercept of 0.958, as shown in Table 7; S-SA-LNP had an average particle size of 81.13 nm, a PDI of 0.159 and an intercept of 0.939, as shown in Table 8; RBD-SA-LNP had an average particle size of 82.74 nm, a PDI of 0.112 and an intercept of 0.960, as shown in Table 9.

**Table 6: Combo A-LNP**

| | Particle size (nm) | Strength (%) | Standard deviation |
|---|---|---|---|
| Peak 1 | 91.43 | 100 | 32.02 |
| Peak 2 | 0 | 0 | 0 |
| Peak 3 | 0 | 0 | 0 |

**Table 7: Combo B-LNP**

| | Particle size (nm) | Strength (%) | Standard deviation |
|---|---|---|---|
| Peak 1 | 91.05 | 100 | 30.06 |
| Peak 2 | 0 | 0 | 0 |
| Peak 3 | 0 | 0 | 0 |

**Table 8: S-SA-LNP**

| | Particle size (nm) | Strength (%) | Standard deviation |
|---|---|---|---|
| Peak 1 | 93.36 | 100 | 33.28 |
| Peak 2 | 0 | 0 | 0 |
| Peak 3 | 0 | 0 | 0 |

**Table 9: RBD-SA-LNP**

| | Particle size (nm) | Strength (%) | Standard deviation |
|---|---|---|---|
| Peak 1 | 92.91 | 100 | 30.56 |
| Peak 2 | 0 | 0 | 0 |
| Peak 3 | 0 | 0 | 0 |

BALB/c female mice aged about 6 weeks were randomized into 5 groups each containing 6 mice. The mice were intramuscularly administered at 5 µg on days 0 and 14. S protein-specific antibody titer was measured on days 14 and 28. On day 28, the mice were sacrificed and the cytokine was measured.

### 3. Results

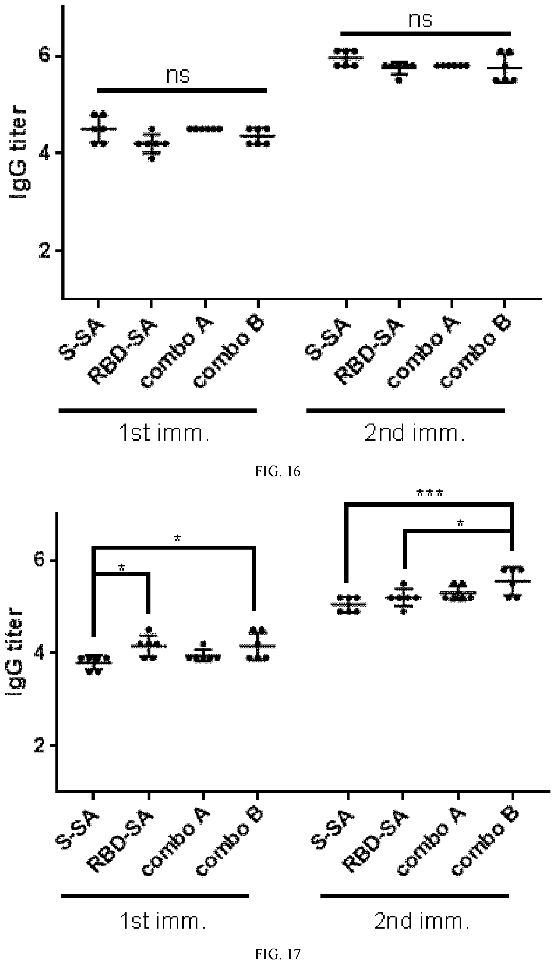

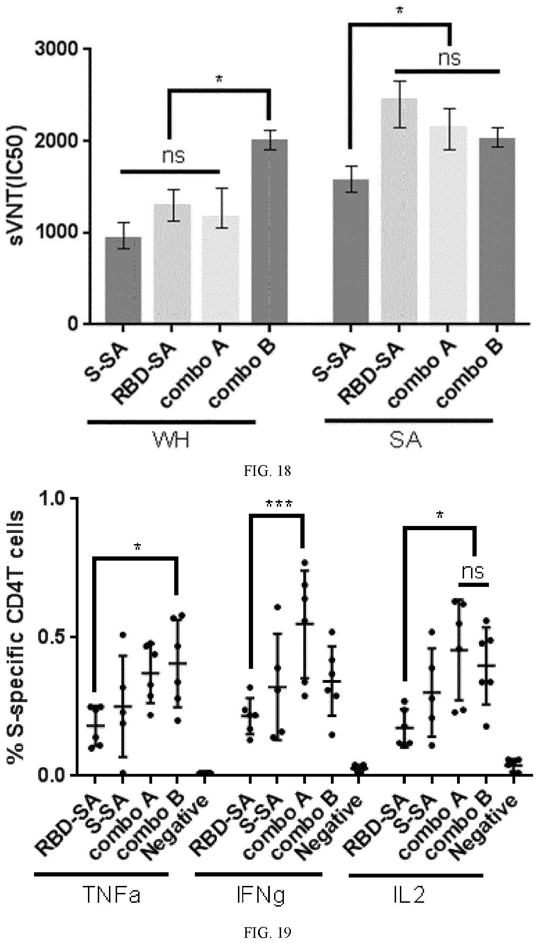

The titers of S protein-specific antibody for 501Y.V2 strain after primary and secondary immunizations are shown in FIG. 16. The groups showed no significant difference. The S protein-specific antibody titers against Wuhan-Hu-1 isolate after the primary and secondary immunizations are shown in FIG. 17. The results showed that the two combined S+RBD immunizations had no significant difference in specific antibody titers, but the specific antibody titers after secondary immunization of the full-length S protein alone and RBD alone were significantly lower than those of the combination of mRNAs derived from different SARS-CoV-2 mutants (combo B). The alternative neutralizing antibody titer (as in FIG. 18, the 4 groups on the left are the alternative neutralizing antibody titer against Wuhan-Hu-1 isolate and the 4 group on the right are the alternative neutralizing antibody titer against the 501Y.V2 strain) also showed that the alternative neutralizing antibody titer against Wuhan-Hu-1 isolate of combo B was significantly higher than the mRNA combination derived from the same SARS-CoV-2 mutant (501Y.V2 strain) (combo A), full-length S protein alone and RBD alone; the alternative neutralizing antibody titer against the 501Y.V2 strain of combo B had no significant difference from combo A and RBD alone, and was significantly higher than full-length S protein alone. The results suggested that the combination of full-length S protein and RBD had humoral immunity advantages over full-length S protein alone, and that mRNA combination derived from different SARS-CoV-2 mutants can provide superior cross-protection in humoral immunity than mRNA combination derived from the same SARS-CoV-2 mutant.

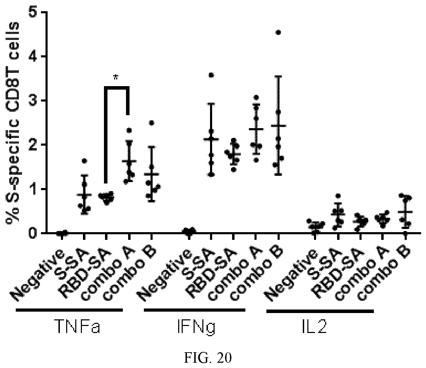

The cellular immune response detection results in the CD4+ T cell Th1 subtype and the CD8+ T cell Th1 subtype are shown in FIGs. 19 and 20. The results suggested that the combination of full-length S protein and RBD has cellular immunity advantages over RBD alone. The superiority of the combined design of full-length S protein and RBD in the aspect of vaccine application is confirmed.

The preferred embodiments of the present invention are described in detail above, which, however, are not intended to limit the present invention. Within the scope of the technical concept of the present invention, various simple modifications can be made to the technical solution of the present invention, all of which will fall within the protection scope of the present invention. In addition, it should be noted that the various specific technical features described in the above specific embodiments can be combined in any suitable manner without contradiction. In order to avoid unnecessary repetition, such combinations will not be illustrated separately.

## Claims

1. An mRNA or mRNA composition, comprising: an mRNA sequence encoding an S protein of SARS-CoV-2 or a variant thereof, and an mRNA sequence encoding an RBD in the S protein or a variant thereof.

2. The mRNA or mRNA composition according to claim 1, wherein the mRNA sequence encoding the S protein of SARS-CoV-2 or the variant thereof and the mRNA sequence encoding the RBD in the S protein or the variant thereof are derived from the same SARS-CoV-2 mutant or different SARS-CoV-2 mutants.

3. The mRNA or mRNA composition according to claim 1 or 2, wherein the S protein or the variant thereof comprises a wild-type full-length S protein or a full-length S protein fixed in a pre-fusion conformation; preferably, the full-length S protein fixed in the pre-fusion conformation comprises a mutation at positions 682RRAR685 and/or a mutation at positions 986KV987, such that the S protein is fixed in the pre-fusion conformation; more preferably, the full-length S protein fixed in the pre-fusion conformation is obtained by mutating 682RRAR685 of the wild-type full-length S protein to GSAG and/or 986KV987 to PP.

4. The mRNA or mRNA composition according to claim 3, wherein the wild-type full-length S protein comprises an amino acid sequence having 70%, 75%, 80%, 85%, 90%, 95% or 99% identity to an amino acid sequence as set forth in SEQ ID NO: 1; the full-length S protein fixed in the pre-fusion conformation comprises an amino acid sequence set forth in SEQ ID NO: 2 or SEQ ID NO: 15, or an amino acid sequence having 70%, 75%, 80%, 85%, 90%, 95% or 99% identity to SEQ ID NO: 2 or 15.

5. The mRNA or mRNA composition according to any one of claims 1-4, wherein the S protein or the variant thereof does not comprise a signal peptide, comprises a signal peptide of the wild-type S protein or comprises a signal peptide of the wild-type S protein and a preceding strong signal peptide; preferably, the strong signal peptide is a signal peptide of tissue plasminogen activator or a signal peptide of serum immunoglobulin E.

6. The mRNA or mRNA composition according to claim 1, wherein the RBD comprises an amino acid sequence set forth in SEQ ID NO: 3 or SEQ ID NO: 13, or an amino acid sequence having 70%, 75%, 80%, 85%, 90%, 95% or 99% identity to SEQ ID NO: 3 or 13.

7. The mRNA or mRNA composition according to claim 1 or 6, wherein the RBD or the variant thereof does not comprise a signal peptide, comprises a signal peptide of the wild-type S protein or comprises a signal peptide of the wild-type S protein and a preceding strong signal peptide; preferably, the strong signal peptide is a signal peptide of tissue plasminogen activator or a signal peptide of serum immunoglobulin E.

8. The mRNA or mRNA composition according to any one of claims 1-7, wherein the mRNA sequence encoding the S protein of SARS-CoV-2 or the variant thereof and the mRNA sequence encoding the RBD in the S protein or the variant thereof are two separate mRNA sequences or are ligated in one mRNA sequence.

9. The mRNA or mRNA composition according to claim 8, wherein the ligation order for the one mRNA sequence from 5' to 3' is: the mRNA sequence encoding the S protein of SARS-CoV-2 or the variant thereof to the mRNA sequence encoding the RBD in the S protein or the variant thereof, or the mRNA sequence encoding the RBD in the S protein or the variant thereof to the mRNA sequence encoding the S protein of SARS-CoV-2 or the variant thereof; preferably, the mRNA sequence encoding the S protein of SARS-CoV-2 or the variant thereof and the mRNA sequence encoding the RBD in the S protein or the variant thereof are ligated via an internal ribosome entry site; the internal ribosome entry site is selected from picornavirus, pestivirus, poliovirus, encephalomyocarditis virus, foot-and-mouth disease virus, hepatitis C virus, classical swine fever virus, mouse leukoma virus, simian immunodeficiency virus and cricket paralysis disease.

10. The mRNA or mRNA composition according to any one of claims 1-9, wherein the mRNA or mRNA composition further comprises a 5' cap, a 5' non-coding region and a polyA tail.

11. The mRNA or mRNA composition according to any one of claims 1-10, wherein the mRNA or mRNA composition further comprises one or a combination of two or more of a 5' conserved sequence element, an RNA replicase coding region, a subgenomic promoter, a 3' conserved sequence element and a 3' non-coding region.

12. The mRNA or mRNA composition according to any one of claims 1-11, wherein the mRNA is a conventional mRNA, a self-amplifying mRNA, or a trans-amplifying mRNA.

13. The mRNA or mRNA composition according to any one of claims 1-12, wherein the mRNA or mRNA composition comprises an mRNA sequence consisting of the mRNA sequence encoding the S protein of SARS-CoV-2 or the variant thereof and the mRNA sequence encoding the RBD in the S protein or the variant thereof selected from any one of the following:
A) consisting of the 5' cap, the 5' non-coding region, the mRNA sequence encoding the S protein of SARS-CoV-2 or the variant thereof, the mRNA sequence encoding the RBD in the S protein or the variant thereof, the 3' non-coding region and the polyA tail;
B) consisting of the 5' cap, the 5' non-coding region, the mRNA sequence encoding the RBD in the S protein or the variant thereof, the mRNA sequence encoding the S protein of SARS-CoV-2 or the variant thereof, the 3' non-coding region and the polyA tail;
C) consisting of the 5' cap, the 5' non-coding region, the mRNA sequence encoding the S protein of SARS-CoV-2 or the variant thereof, the internal ribosome entry site (IRES), the mRNA sequence encoding the RBD in the S protein or the variant thereof, the 3' non-coding region and the polyA tail;
D) consisting of the 5' cap, the 5' non-coding region, the mRNA sequence encoding the RBD in the S protein or the variant thereof, the IRES, the mRNA sequence encoding the S protein of SARS-CoV-2 or the variant thereof, the 3' non-coding region and the polyA tail;
E) consisting of the 5' cap, the 5' conserved sequence element, the RNA replicase coding region, the subgenomic promoter, the mRNA sequence encoding the RBD in the S protein or the variant thereof, the mRNA sequence encoding the S protein of SARS-CoV-2 or the variant thereof, the 3' conserved sequence element and the polyA tail;
F) consisting of the 5' cap, the 5' conserved sequence element, the RNA replicase coding region, the subgenomic promoter, the mRNA sequence encoding the S protein of SARS-CoV-2 or the variant thereof, the mRNA sequence encoding the RBD in the S protein or the variant thereof, the 3' conserved sequence element and the polyA tail;
G) consisting of the 5' cap, the 5' conserved sequence element, the RNA replicase coding region, the subgenomic promoter, the mRNA sequence encoding the RBD in the S protein or the variant thereof, the IRES, the mRNA sequence encoding the S protein of SARS-CoV-2 or the variant thereof, the 3' conserved sequence element and the polyA tail; and
H) consisting of the 5' cap, the 5' conserved sequence element, the RNA replicase coding region, the subgenomic promoter, the mRNA sequence encoding the S protein of SARS-CoV-2 or the variant thereof, the IRES, the mRNA sequence encoding the RBD in the S protein or the variant thereof, the 3' conserved sequence element and the polyA tail.

14. The mRNA or mRNA composition according to any one of claims 1-12, wherein the mRNA or mRNA composition comprises a combination of two mRNA sequences selected from any one of the following:
a) an mRNA consisting of the 5' cap, the 5' non-coding region, the mRNA sequence encoding the S protein of SARS-CoV-2 or the variant thereof, the 3' non-coding region and the polyA tail, combined with an mRNA consisting of the 5' cap, the 5' non-coding region, the mRNA sequence encoding the RBD in the S protein or the variant thereof, the 3' non-coding region and the polyA tail;
b) an mRNA consisting of the 5' cap, the 5' conserved sequence element, the RNA replicase coding region, the subgenomic promoter, the mRNA sequence encoding the RBD in the S protein or the variant thereof, the 3' conserved sequence element and the polyA tail, combined with an mRNA consisting of the 5' cap, the 5' conserved sequence element, the RNA replicase coding region, the subgenomic promoter, the mRNA sequence encoding the S protein of SARS-CoV-2 or the variant thereof, the 3' conserved sequence element and the polyA tail;
c) an mRNA consisting of the 5' cap, the 5' conserved sequence element, the subgenomic promoter, the mRNA sequence encoding the S protein of SARS-CoV-2 or the variant thereof, the IRES, the mRNA sequence encoding the RBD in the S protein or the variant thereof, the 3' conserved sequence element and the polyA tail, combined with an mRNA consisting of the 5' cap, the 5' non-coding region, the RNA replicase coding region, the 3' non-coding region and the polyA tail;
d) an mRNA consisting of the 5' cap, the 5' conserved sequence element, the subgenomic promoter, the mRNA sequence encoding the RBD in the S protein or the variant thereof, the IRES, the mRNA sequence encoding the S protein of SARS-CoV-2 or the variant thereof, the 3' conserved sequence element and the polyA tail, combined with an mRNA consisting of the 5' cap, the 5' non-coding region, the RNA replicase coding region, the 3' non-coding region and the polyA tail;
e) an mRNA consisting of the 5' cap, the 5' conserved sequence element, the subgenomic promoter, the mRNA sequence encoding the S protein of SARS-CoV-2 or the variant thereof, the mRNA sequence encoding the RBD in the S protein or the variant thereof, the 3' conserved sequence element and the polyA tail, combined with an mRNA consisting of the 5' cap, the 5' non-coding region, the RNA replicase coding region, the 3' non-coding region and the polyA tail; and
f) an mRNA consisting of the 5' cap, the 5' conserved sequence element, the subgenomic promoter, the mRNA sequence encoding the RBD in the S protein or the variant thereof, the mRNA sequence encoding the S protein of SARS-CoV-2 or the variant thereof, the 3' conserved sequence element and the polyA tail, combined with an mRNA consisting of the 5' cap, the 5' non-coding region, the RNA replicase coding region, the 3' non-coding region and the polyA tail.

15. The mRNA or mRNA composition according to claim 13 or 14, wherein the RNA replicase coding region is selected from alphavirus, picornavirus, flavivirus, paramyxovirus and calicivirus.

16. An mRNA vaccine comprising the mRNA or mRNA composition according to any one of claims 1-15.

17. The mRNA vaccine according to claim 16, wherein in the mRNA vaccine, the mass ratio of the mRNA sequence encoding the S protein of SARS-CoV-2 or the variant thereof to the mRNA sequence encoding the RBD in the S protein or the variant thereof is (1-5):(1-5).

18. The mRNA vaccine according to claim 16 or 17, wherein the mRNA vaccine further comprises a cationic or polycationic compound.

19. The mRNA vaccine according to any one of claims 16-18, wherein the mRNA vaccine further comprises a lipid.

20. The mRNA vaccine according to any one of claims 16-19, wherein the mRNA vaccine is a liposome, a lipid complex or a lipid nanoparticle.

21. A method for preparing an mRNA vaccine, comprising: mixing the mRNA or mRNA composition according to any one of claims 1-15 with a cationic or polycationic compound, and packaging the mixture with a lipid.

22. Use of the mRNA or mRNA composition according to any one of claims 1-15 or the mRNA vaccine according to any one of claims 16-20, wherein the use comprises:
A) use in preparing a medicament for preventing and/or treating a disease caused by SARS-CoV-2 infection; or
B) use in preparing a medicament for resisting SARS-CoV-2 infection;
preferably, the disease caused by SARS-CoV-2 infection is COVID-19.

23. A method for screening an antibody, comprising: administering to an individual the mRNA or mRNA composition according to any one of claims 1-15, or the mRNA vaccine according to any one of claims 16-20.

24. A method for inducing a neutralizing antigen-specific immune response in an individual, comprising administering to the individual the mRNA or mRNA composition according to any one of claims 1-15, or the mRNA vaccine according to any one of claims 16-20.
